(19) Europäisches Patentamt
European Patent Office
Office européen des brevets

(11) **EP 3 269 302 A1**

(12) **EUROPEAN PATENT APPLICATION**
published in accordance with Art. 153(4) EPC

(43) Date of publication:
**17.01.2018 Bulletin 2018/03**

(51) Int Cl.:
***A61B 5/11*** *(2006.01)*

(21) Application number: **16761374.4**

(86) International application number:
**PCT/JP2016/052296**

(22) Date of filing: **27.01.2016**

(87) International publication number:
**WO 2016/143402 (15.09.2016 Gazette 2016/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **11.03.2015 JP 2015048172**

(71) Applicant: **Sony Corporation
Tokyo 108-0075 (JP)**

(72) Inventors:
• **MURAKOSHI, Sho
Tokyo 108-0075 (JP)**
• **YAMASHITA, Kosei
Tokyo 108-0075 (JP)**
• **AOKI, Suguru
Tokyo 108-0075 (JP)**

(74) Representative: **MFG Patentanwälte
Meyer-Wildhagen Meggle-Freund
Gerhard PartG mbB
Amalienstraße 62
80799 München (DE)**

(54) **INFORMATION PROCESSING APPARATUS AND INFORMATION PROCESSING METHOD**

(57) [Object]
To provide an information processing device and information processing method, capable of obtaining useful information in which the attitude of a target person is taken into consideration from sensor information.
[Solution]
The information processing device includes: an acquisition unit configured to acquire information indicating a measurement result from a plurality of sensor devices configured to measure a pressure distribution of an attached part of a body of a user; an estimation unit configured to estimate arrangement of a plurality of parts to which the sensor devices are attached on the basis of a captured image including the plurality of parts; and a calculation unit configured to calculate information on the plurality of parts from the information indicating the measurement result acquired by the acquisition unit on the basis of the arrangement of the plurality of parts estimated by the estimation unit.

**FIG. 1**

**Description**

Technical Field

The present disclosure relates to an information processing device and an information processing method.

Background Art

In recent years, attempts have been made to apply information processing technology in various fields. One example is the technique of visualizing the movement of the player's body in the field of sports. It is possible for the player to check whether the action corresponding to the sport is performed smoothly by measuring and recording the movement of his/her body using various sensor devices. This makes it possible for the player to improve easily his/her posture or the like with reference to the visualized body movement.

Techniques for visualizing the body's movement have various approaches including motion capture. In one example, Patent Literature 1 below discloses a technique of calculating coordinates in the three-dimensional space of a marker affixed to a target person to calculate a value indicating the attitude of the target person on the basis of a range image. This range image is obtained by calculating a distance depending on a time lag until the reflected wave of the light used to irradiate the target person is received.

Citation List

Patent Literature

Patent Literature 1: JP 2012-120648A

Disclosure of Invention

Technical Problem

However, the technique disclosed in Patent Literature 1 is only able to calculate information indicating the attitude of the target person. It is considerable that sensor information detected by various sensor devices for the target person, in some cases, has different meanings depending on the attitude of the target person at the time when detection is performed. In view of this, the present disclosure provides a novel and improved information processing device and information processing method, capable of obtaining useful information in which the attitude of a target person is taken into consideration from sensor information.

Solution to Problem

According to the present disclosure, there is provided an information processing device including an acquisition unit configured to acquire information indicating a measurement result from a plurality of sensor devices configured to measure a pressure distribution of an attached part of a body of a user, an estimation unit configured to estimate arrangement of a plurality of parts to which the sensor devices are attached on the basis of a captured image including the plurality of parts, and a calculation unit configured to calculate information on the plurality of parts from the information indicating the measurement result acquired by the acquisition unit on the basis of the arrangement of the plurality of parts estimated by the estimation unit.

Furthermore, according to the present disclosure, there is provided an information processing method executed by a processor, the method including acquiring information indicating a measurement result from a plurality of sensor devices configured to measure a pressure distribution of an attached part of a body of a user, estimating arrangement of a plurality of parts to which the sensor devices are attached on the basis of a captured image including the plurality of parts, and calculating information on the plurality of parts from the acquired information indicating the measurement result on the basis of the estimated arrangement of the plurality of parts.

Advantageous Effects of Invention

According to the present disclosure as described above, it is possible to obtain useful information in which the attitude of the target person is taken into consideration from the sensor information. Note that the effects described above are not necessarily limitative. With or in the place of the above effects, there may be achieved any one of the effects described in this specification or other effects that may be grasped from this specification.

Brief Description of Drawings

[FIG. 1] FIG. 1 is a diagram illustrated to describe an overview of ZMP.

[FIG. 2] FIG. 2 is a diagram illustrated to describe a method of calculating ZMP of one foot.

[FIG. 3] FIG. 3 is a diagram illustrated to describe a method of calculating ZMP of both feet.

[FIG. 4] FIG. 4 is a block diagram illustrating an example of a logical configuration of a sensing system according to the present embodiment.

[FIG. 5] FIG. 5 is a diagram illustrated to describe an arrangement estimation function using an AR marker according to the present embodiment.

[FIG. 6] FIG. 6 is a diagram illustrated to describe an arrangement estimation function using an AR marker according to the present embodiment.

[FIG. 7] FIG. 7 is a diagram illustrated to describe an arrangement estimation function using an AR marker according to the present embodiment.

[FIG. 8] FIG. 8 is a diagram illustrated to describe an arrangement estimation function using an AR marker according to the present embodiment.

[FIG. 9] FIG. 9 is a diagram illustrated to describe an arrangement estimation function using an AR marker according to the present embodiment.

[FIG. 10] FIG. 10 is a flowchart illustrating an example of the procedure of ZMP calculation processing of both feet executed in the sensing system according to the present embodiment.

[FIG. 11] FIG. 11 is a block diagram illustrating an example of a hardware configuration of an information processing device according to the present embodiment.

Mode(s) for Carrying Out the Invention

Hereinafter, (a) preferred embodiment(s) of the present disclosure will be described in detail with reference to the appended drawings. In this specification and the appended drawings, structural elements that have substantially the same function and structure are denoted with the same reference numerals, and repeated description of these structural elements is omitted.

Further, there is a case in which elements having substantially the same function are discriminated by affixing different alphabets to the back of the same reference numeral in the present specification and drawings. In one example, elements having substantially the same functional configuration are discriminated as sensor devices 200A, 200B, and 200C as necessary. However, when there is no need to discriminate particularly between a plurality of elements having substantially the same functional configuration, only the same reference numeral is affixed. In one example, when there is no need to discriminate particularly between the sensor devices 200A, 200B, and 200C, these sensor devices are referred to collectively as a sensor device 200.

Moreover, the description will be given in the following order.

1. Overview
1.1. ZMP
1.2. Technical challenges
2. Configuration example
3. Function details
3.1. Arrangement estimation function
3.2. Update function
3.3. Information acquisition function
3.4. ZMP calculation function
4. Operation processing example
5. Hardware configuration example
6. Summary

<<1. Overview>>

<1.1. ZMP>

In one embodiment of the present disclosure, we will focus on zero moment point (ZMP) as an example of useful information obtained from sensor information. The ZMP is the center of pressure of the ground reaction force.

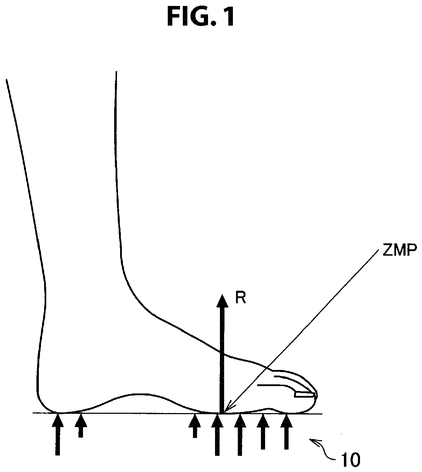

The ZMP is described now in detail with reference to FIGS. 1 to 3.

FIG. 1 is a diagram illustrated to describe the overview of ZMP. In FIG. 1, reference sign 10 is a vector indicating the load of the body applied to the sole of the foot. As indicated by the reference sign 10, the loads of the body having the same sign are applied to the entire surface of the sole of the foot contacting the ground. These loads can be grouped as an equivalent force vector R acting on one point existing inside the sole surface of the foot. The point of action through which this force vector R passes is the ZMP.

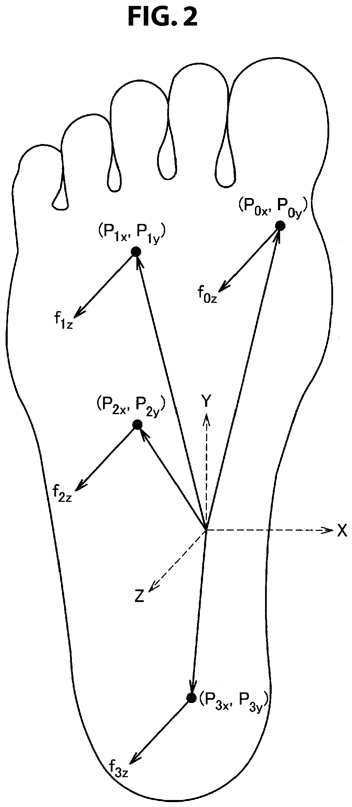

FIG. 2 is a diagram illustrated to describe a method of calculating ZMP of one foot. As illustrated in FIG. 2, the position vector at any position on the sole of the foot is set to $(P_{jx}, P_{jy})$, and the Z component of the force applied from the ground surface at that position is set to $f_{jz}$. The position vector $(P_x, P_y)$ of ZMP of one foot is calculated by the following formula.

[Math. 1]

$$P_x = \sum P_{jx} f_{jz} \Big/ \sum f_{jz}$$
$$P_y = \sum P_{jy} f_{jz} \Big/ \sum f_{jz} \qquad \cdots \text{Formula (1)}$$

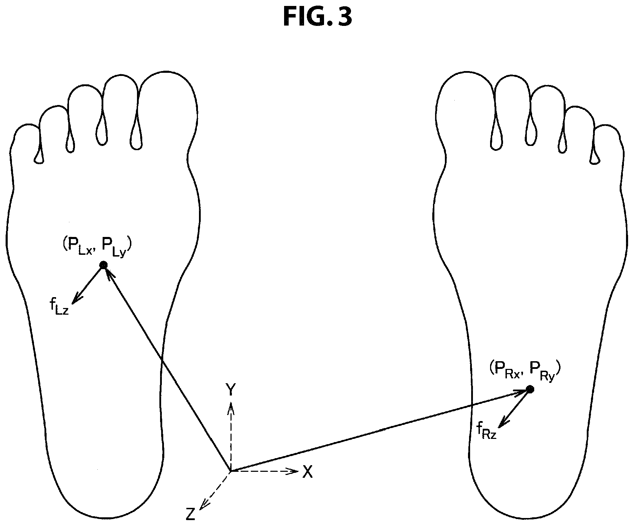

FIG. 3 is a diagram illustrated to describe a method of calculating ZMP of both feet. As illustrated in FIG. 3, the position vector of ZMP of the right foot is set to $(P_{Rx}, P_{Ry})$, and the Z component of the force applied from the ground surface at that position is set to $f_{Rz}$. In addition, the position vector of ZMP of the left foot is set to $(P_{Lx}, P_{Ly})$, and the Z component of the force applied from the ground surface at that position is set to $f_{Lz}$. The position vector $(P_x, P_y)$ of ZMP of both feet is calculated by the following formula.

[Math. 2]

$$P_x = \left(P_{Rx} f_{Rz} + P_{Lx} f_{Lz}\right) \Big/ \left(f_{Rz} + f_{Lz}\right)$$
$$P_y = \left(P_{Ry} f_{Rz} + P_{Ly} f_{Lz}\right) \Big/ \left(f_{Rz} + f_{Lz}\right) \qquad \cdots \text{Formula (2)}$$

<1.2. Technical challenges>

In some cases, it is important for a player to recognize his/her weight shift to support game improvement in the field of sports. In one example, the player recognizes the timing of action and weight shift and the amount of shift in performing the weight shift in a particular action such as golf swing, thereby achieving faster improvement.

A force platform is one example of instruments capable of measuring such weight shift. The force platform has a flat plate on which a person can ride and measures the ground reaction force to an object placed on the flat plate. However, in some cases, the force platform has restrictions including a limited range of measurable target to an object within a limited range of the flat plate or to the action performed within the range, an installation location limited to indoors, or necessity of installing it horizontally.

An insole-type pressure distribution sensor is one example of another instrument. The insole-type pressure distribution sensor has one or more pressure sensors arranged on the insole and can measure the distribution of pressure applied to the sole of the user wearing the sensor. The insole-type pressure distribution sensor does not have the restrictions described above, and so it can be said that it has higher convenience than the force platform. However, the arrangement relationship (position vector) of both feet is unknown in measurement using the insole-type sensor, and so it is difficult to calculate the ZMP of both feet using Formula (2) described above.

Thus, in view of the above circumstances, a sensing system according to an embodiment of the present disclosure has been developed. It is possible for the sensing system according to the present embodiment to estimate the user's attitude and obtain useful information in which the user's attitude is taken into consideration from sensor information. Specifically, it is possible for the sensing system according to the present embodiment to estimate the attitude of both feet of the user and calculate the ZMP of both feet on the basis of the pressure distribution obtained from each of the insole-type sensors of both feet.

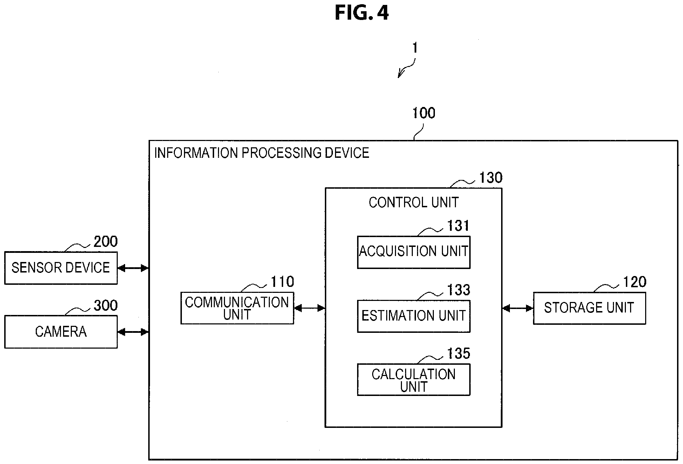

The overview of the sensing system according to the present embodiment is described above. The sensing system according to the present embodiment is described now in more detail with reference to FIGS. 4 to 11.

<<2. Configuration example>>

FIG. 4 is a block diagram illustrating an example of a logical configuration of a sensing system 1 according to the present embodiment. As illustrated in FIG. 4, the sensing system 1 according to the present embodiment is configured to include an information processing device 100, a sensor device 200, and a camera 300.

The sensor device 200 has a function of measuring information on a target object. In one example, the sensor device 200 is implemented as a pressure distribution sensor that measures a pressure distribution of an attached part of the body of the user. In one example, the sensor device 200 may be implemented as the insole-type pressure distribution sensor described above, which is attached to the sole of both feet of the user. The ZMP of both feet is useful for, in one example, the swing motion in golf. In the case of using the insole-type pressure distribution sensor, the information processing device 100 can calculate the ZMP of both feet for games, such as golf and skiing, performing while wearing a tool such as shoes and skis. In addition, the sensor device 200 may be implemented as a globe-type pressure distribution sensor attached to both hands of the user. The ZMP of both hands is useful for, in one example, the handstand motion of a gymnast. Alternatively, the sensor device 200 may be implemented as an inertial sensor such as acceleration sensors and gyro sensors, a biological sensor such as myoelectric sensors, neural sensors, pulse sensors, and body temperature sensors, a vibration sensor, a geomagnetic sensor, or the like. The following description will be given on the assumption that the insole-type pressure distribution sensor is used as an example.

The camera 300 has a function of capturing an image (still image or moving image). The camera 300 is configured to include a lens system, a driving system, a solid-state image sensor array, or the like. The lens system is composed of an image capturing lens, a diaphragm, a zoom lens, a focus lens, or the like. The driving system causes the lens system to perform a focusing operation and a zooming operation. The solid-state image sensor array photoelectrically converts image-capturing light obtained by the lens system to generate an image-capturing signal. The solid-state image sensor array may be implemented as, in one example, a charge coupled device (CCD) sensor array or a complementary metal oxide semiconductor (CMOS) sensor array. The camera 300 outputs data of a captured image taken as a digital signal to the information processing device 100. The camera 300 and the information processing device 100 may communicate with each other wirelessly or wired.

The information processing device 100 calculates useful information in which the user's attitude is taken into consideration from the sensor information obtained from the plurality of sensor devices 200. As illustrated in FIG. 4, the information processing device 100 is configured to include a communication unit 110, a storage unit 120, and a control unit 130.

The communication unit 110 is a communication module that transmits and receives data to and from an external device. In one example, the communication unit 110 transmits and receives data to and from the sensor device 200 and the camera 300. The communication unit 110 directly communicates, or indirectly communicates via another communication node such as a network access point, with the sensor device 200 and the camera 300 using a communication scheme such as a wireless local area network (LAN), Wireless Fidelity (Wi-Fi, registered trademark), infrared communication, Bluetooth (registered trademark). The communication unit 110 may perform wired communication with an external device using a communication scheme such as a wired LAN.

The storage unit 120 is a unit that records data on and reproduces data from a predetermined recording medium. In one example, the storage unit 120 stores the sensor information received from the plurality of sensor devices 200.

The control unit 130 functions as an arithmetic processing unit and a control unit, and controls the overall operation in the information processing device 100 in accordance with various programs. As illustrated in FIG. 4, the control unit 130 functions as an acquisition unit 131, an estimation unit 133, and a calculation unit 135.

The acquisition unit 131 has a function of acquiring the sensor information from the plurality of sensor devices 200. The estimation unit 133 has a function of estimating the arrangement of a plurality of parts to which the sensor devices 200 are attached. The calculation unit 135 has a function of calculating useful information in which an estimation result obtained by the estimation unit 133 is taken into consideration from the sensor information acquired by the acquisition unit 131.

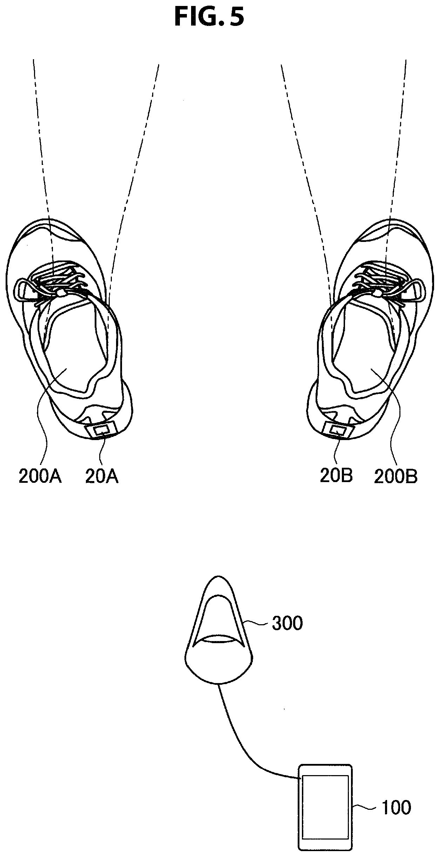

The configuration example of the sensing system 1 according to the present embodiment is described above. Subsequently, the functions of the sensing system 1 according to the present embodiment are described in detail with reference to FIGS. 5 to 9.

<<3. Function details>>

<3.1. Arrangement estimation function>

The information processing device 100 (e.g., the estimation unit 133) estimates the arrangement of the sensor device 200. In the present embodiment, the estimation unit 133 estimates the arrangement of the plurality of sensor devices 200 that are spaced apart from each other and are able to dynamically change the arrangement relationship, like the insole-type sensor device 200.

Specifically, first, the estimation unit 133 estimates the arrangement of the plurality of parts to which the sensor devices 200 are attached on the basis of the captured image including the plurality of parts. In one example, the estimation unit 133 estimates the arrangement of both feet on the basis of the captured image obtained by capturing the both feet to which the insole-type sensor device 200 is attached by the camera 300. Then, the estimation unit 133 estimates the arrangement of the plurality of sensor devices 200 on the basis of the estimation result of the arrangement of the plurality of parts. In one example, the estimation unit 133 estimates a position vector of each of the pressure sensors provided on the insole by incorporating the relative arrangement relationship between the foot and the insole-type sensor device 200 into the angle and the distance between the estimated right foot and the estimated left foot.

Here, it is considerable that there are various arrangement estimation methods based on the captured image.

In one example, the estimation unit 133 may estimate the arrangement of the plurality of parts included in the captured image by performing image recognition on the plurality of parts. Specifically, the estimation unit 133 may estimate the arrangement of the both feet by previously acquiring design information such as the shape, pattern, size, and the like of the shoes and performing image recognition based on the design information with respect to the captured image in which the shoes of both feet are captured. The estimation unit 133 may acquire the design information from a server or the like, or may acquire the design information by performing image recognition on an information code such as QR code (registered trademark) provided in shoes.

In one example, the estimation unit 133 may estimate the arrangement of the plurality of parts included in the captured image by estimating the position and attitude of each of markers provided at the plurality of parts. Specifically, the estimation unit 133 may estimate the arrangement of both feet by estimating the position and attitude of an AR marker provided at each of the shoes of both feet on the basis of the captured image in which the augmented reality (AR) marker is photographed. In one example, the estimation unit 133 may previously acquire design information indicating the shape of the shoe, the shape of the AR marker, the position and angle at which the AR marker is attached, or the like. Then, the estimation unit 133 estimates the position and attitude of the AR marker, and calculates the arrangement of both feet from the estimation result using the design information. The estimation unit 133 may acquire the design information from a server or the like, or may acquire the design information by performing image recognition on an information code such as QR code or the like provided in a shoe. In addition, the estimation unit 133 may acquire information indicating a portion of the shoe in which the AR marker is provided from the server or the like. This reduces the load for recognition of the AR marker.

An example of an algorithm for estimating the position and attitude of the AR marker is disclosed in, for example, "An Augmented Reality System and its Calibration based on Marker Tracking" in TVRSJ, Vol. 4, No. 4, 1999, by Hirokazu Kato, Mark Billinghurst, Koichi Asano, and Keihachiro Tachibana. An example of the algorithm is described below with reference to FIGS. 5 to 9.

(Example of algorithm)

FIGS. 5 to 9 are diagrams illustrated to describe an arrangement estimation function using the AR marker according to the present embodiment.

FIG. 5 illustrates an example of settings of the sensing system 1. In the example illustrated in FIG. 5, the information processing device 100 is a smartphone, the sensor devices 200A and 200B are insole-type pressure distribution sensors, and the camera 300 captures the user's feet from the back of the user. As illustrated in FIG. 5, each of AR markers 20A and 20B is provided at the heel portion of the shoes worn by the user on both feet, and the camera 300 is capable of capturing the AR markers 20A and 20B. Moreover, in addition to the example illustrated in FIG. 5, in one example, a camera for photographing the tops of both feet from above may be provided, and an AR marker may be provided on the tops of the both feet.

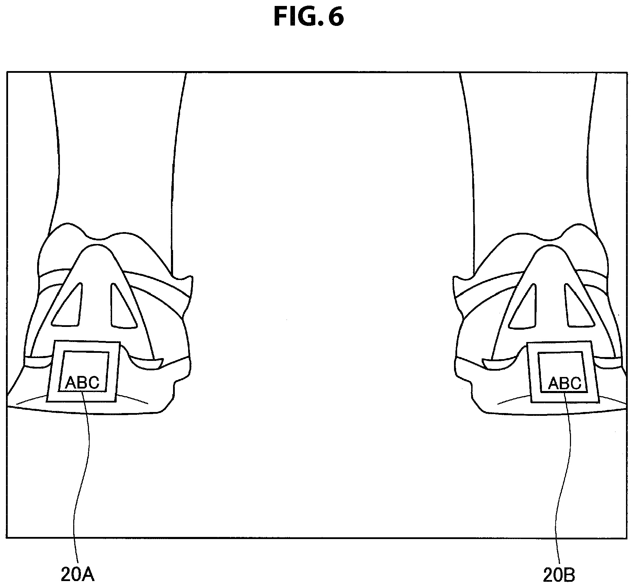

FIG. 6 illustrates an example of a captured image captured by the camera 300. As illustrated in FIG. 6, the captured image captured by the camera 300 includes the AR markers 20A and 20B. The estimation unit 133 estimates the position and attitude of each of the AR markers 20A and 20B from the captured image illustrated as an example in FIG. 6 by using the algorithm described below.

In this algorithm, four coordinate systems, that is, a marker coordinate system (3D), a camera coordinate system (3D), an ideal screen coordinate system (2D), and an observation screen coordinate system (2D) are used. The marker coordinate system is a coordinate system used in representing a virtual object. The camera coordinate system is a coordinate system in which a focal position is the origin, the direction perpendicular to an image plane is Z axis, and the directions parallel to the x and y axes of the image are X and Y axes, respectively. Moreover, a point represented in the marker coordinate system can be converted into the camera coordinate system by rotation and translation. The ideal screen coordinate system is a coordinate system of a projected image plane. The observation screen coordinate system is a coordinate system of an actual camera image and is the coordinate system in which the distortion of a wide-angle lens is taken into consideration from the ideal screen coordinate system. The ideal screen coordinate system and the camera coordinate system can be interconverted using a perspective transformation model. The ideal screen coordinate system and the marker coordinate system can also be interconverted using the perspective transformation model.

In this algorithm, a matrix for coordinate transformation from the marker coordinate system (3D) to the camera coordinate system (3D) is obtained. This coordinate transformation matrix is composed of a rotational component and a translational component.

The estimation unit 133 previously obtains a parameter of the perspective transformation model of the ideal screen coordinate system and the camera coordinate system by calibration.

If a camera image is acquired, the estimation unit 133 corrects the distortion from the camera image (observation screen coordinate system) and obtains a vertex position of the marker on the ideal screen coordinate system. Specifically, the estimation unit 133 obtains the vertex position by converting the marker into a binary code (black and white image) and by detecting the outline of the marker.

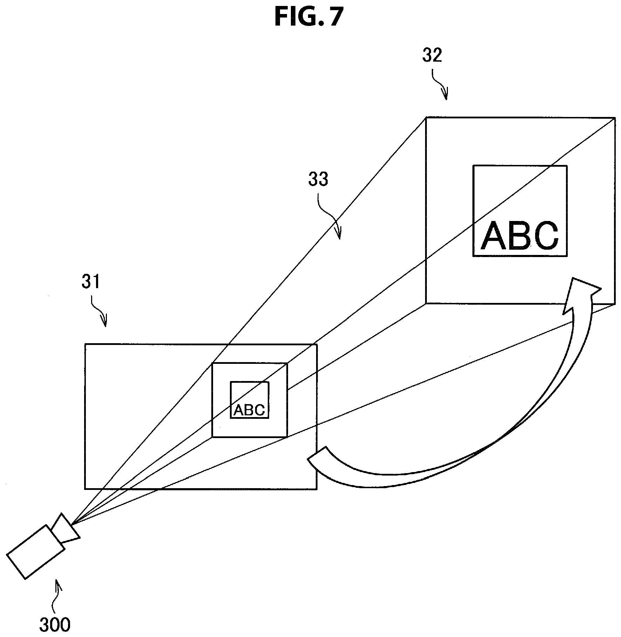

Subsequently, the estimation unit 133 maps the vertex position of the marker on the ideal screen coordinate system to the camera coordinate system by using the previously calculated perspective transformation model. More specifically, as illustrated in FIG. 7, the estimation unit 133 extends the four sides of the marker projected onto the projection surface 31 in the projection direction of the camera 300 to create a plane 32. Then, the estimation unit 133 creates four planes 33 by connecting these sides with the optical center of the camera using an internal parameter of the camera obtained by the camera calibration performed previously.

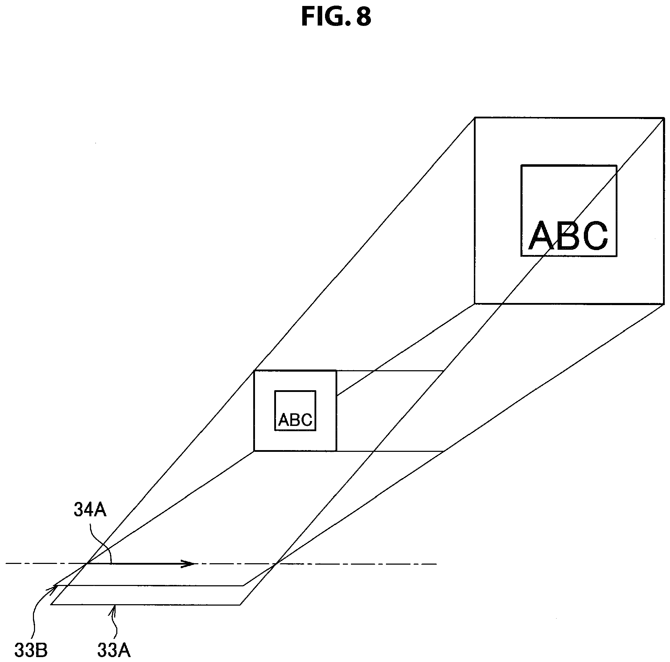

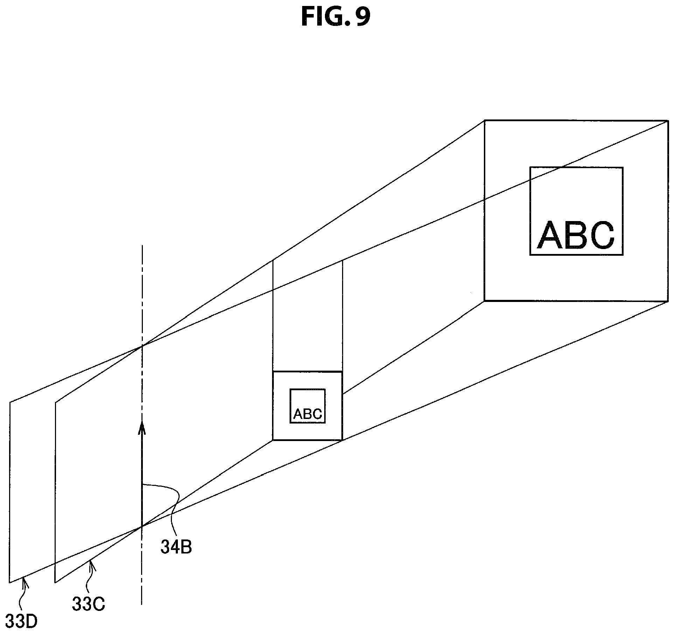

Then, the estimation unit 133 obtains an intersection vector of planes pacing each other. Specifically, as illustrated in FIG. 8, the estimation unit 133 obtains an intersection vector 34A of planes 33A and 33B facing each other. In addition, as illustrated in FIG. 9, the estimation unit 133 obtains an intersection vector 34B of planes 33C and 33D facing each other. Furthermore, the estimation unit 133 calculates a cross product from the two intersection vectors. This allows the rotational component of the marker to be obtained.

Next, the estimation unit 133 obtains the position in the marker coordinate system from the position of the four vertices of the marker in the image coordinate system and the size of the marker. Then, the estimation unit 133 obtains information on the translational component of the marker from the position of the marker in the marker coordinate system, information on the rotational component, and information on the calibration.

The processing described above allows the estimation unit 133 to obtain the calibration information and the information on the rotational and translational components of the marker. The estimation unit 133 is capable of obtaining the position and attitude of the marker in the camera coordinate system from the positional information of the marker on the ideal screen coordinate system by using these pieces of information.

<3.2. Update function>

The information processing device 100 (e.g., the estimation unit 133) may update the estimation result by sequentially estimating the arrangement of the plurality of parts to which the sensor devices 200 are attached. In one example, the estimation unit 133 may repeatedly estimate the arrangement depending on the action of a plurality of parts. In one example, the estimation unit 133 repeatedly estimates the arrangement of both feet of the running user from the images of the user continuously captured by the mechanism in which the camera 300 moves in parallel with the running user.

This makes it possible for the sensing system 1 to acquire the arrangement of both feet of the moving user in real time. In one example, the sensing system 1 can continuously calculate the ZMP of both feet for the user who moves around a wider range than the flat plate of the force platform. In addition, the sensing system 1 can calculate information on the length of stride or the like from the width of both feet of the running user that are landed.

<3.3. Information acquisition function>

(Information acquisition function)

The information processing device 100 (e.g., the acquisition unit 131) acquires the sensor information from the plurality of sensor devices 200.

In one example, the acquisition unit 131 may acquire the sensor information transmitted from the sensor device 200. In one example, the sensor device 200 has a communication interface and transmits the sensor information to the information processing device 100 using wireless or wired communication. Then, the information processing device 100 acquires the sensor information transmitted from the sensor device 200 via the communication unit 110.

In one example, the acquisition unit 131 may acquire the sensor information from display contents displayed on a display device provided at the plurality of parts included in the captured image. In one example, a display device such as electronic paper for displaying an information code representing the sensor information is formed on the heel portion, instep portion, or the like of the shoe, and the insole-type sensor device 200 causes the sensor information to be displayed on the display device. Then, the acquisition unit 131 acquires the sensor information represented in the information code by performing image recognition on the captured image in which the display device is captured. In this case, the sensor device 200 may not necessarily include a communication interface, and the information processing device 100 may not necessarily include the communication unit 110.

<3.4. ZMP calculation function>

The information processing device 100 (e.g., the calculation unit 135) calculates information on the plurality of parts from the sensor information acquired by the acquisition unit 131 on the basis of the arrangement of the plurality of parts estimated by the estimation unit 133. In one example, the calculation unit 135 calculates information on all the plurality of parts to which the sensor devices 200 are attached from the plurality pieces of sensor information on the basis of the arrangement of the sensor device 200. An example of such information includes ZMP. For the insole-type sensor device 200, the calculation unit 135 calculates ZMP of both feet by substituting the position vector of the pressure sensor arranged on the insole estimated by the estimation unit 133 and the sensor information acquired by the acquisition unit 131 into the above Formulas (1) and (2).

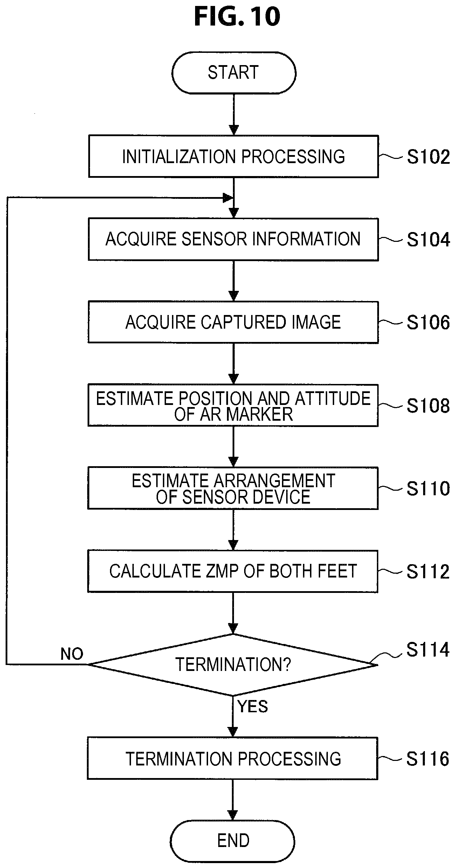

The functions of the sensing system 1 according to the present embodiment are described in detail above. Next, an operation processing example of the sensing system 1 according to the present embodiment is described with reference to FIG. 10.

<<4. Operation processing example>>

FIG. 10 is a flowchart illustrating an example of the procedure of ZMP calculation processing of both feet executed in the sensing system 1 according to the present embodiment.

First, in step S102, the sensing system 1 performs initialization processing. In one example, the sensing system 1 starts settings of allowing the information processing device 100 to acquire a captured image from the camera 300, settings of an internal parameter of the camera 300, load of AR markers, and capturing an image by the camera 300.

Next, in step S104, the sensing system 1 acquires sensor information. In one example, the information processing device 100 receives the pressure distribution from the insole-type sensor device 200 attached to both feet of the user.

Next, in step S106, the sensing system 1 acquires a captured image. In one example, the camera 300 provided in the back of the user captures an image including the AR markers provided at the heel portions of the shoes worn by the user on both feet, and transmits it to the information processing device 100.

Next, in step S108, the sensing system 1 estimates the position and attitude of the AR marker. In one example, the information processing device 100 estimates the position and attitude of each of the AR markers provided at the heel portions of both feet using the algorithm described above.

Next, in step S110, the sensing system 1 estimates the arrangement of the sensor device 200. In one example, the information processing device 100 estimates the position vector of each of the pressure sensors provided on the insole on the basis of the position and attitude of the AR marker.

Then, in step S112, the sensing system 1 calculates the ZMP of both feet. In one example, the information processing device 100 calculates the ZMP of both feet by substituting the estimated position vector of the pressure sensor and the acquired sensor information into the Formulas (1) and (2).

The steps S104 to S112 are repeated until termination is made (NO in step S114). If the termination is made (YES in step S114), the sensing system 1 performs termination processing in step S116. In one example, the sensing system 1 performs photographing end processing, cleanup processing, and the like of the camera 300.

<<5. Hardware configuration example>>

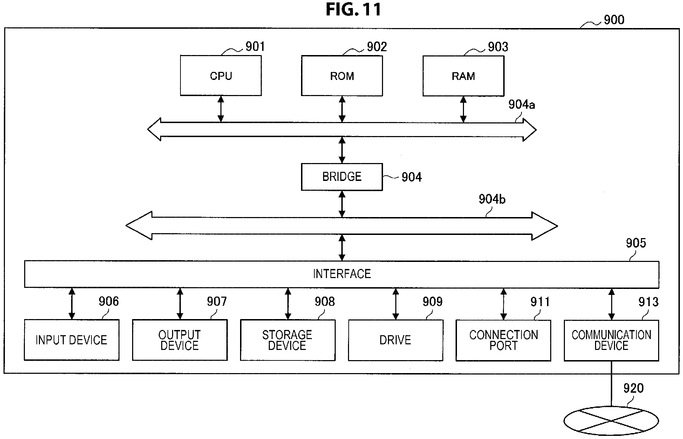

Finally, a hardware configuration of an information processing device according to the present embodiment will be described with reference to FIG. 11. FIG. 11 is a block diagram illustrating an example of the hardware configuration of the information processing device according to the present embodiment. Moreover, the information processing device 900 illustrated in FIG. 11 may be implemented, in one example, as the information processing device 100 illustrated in FIG. 4. The information processing performed by the information processing device 100 according to the present embodiment is achieved by cooperation of software and hardware described below.

As illustrated in FIG. 8, the information processing device 900 is configured to include a central processing unit (CPU) 901, a read only memory (ROM) 902, a random access memory (RAM) 903, and a host bus 904a. In addition, the information processing device 900 is configured to include a bridge 904, an external bus 904b, an interface 905, an input device 906, an output device 907, a storage device 908, a drive 909, a connection port 911, and a communication device 913. The information processing device 900 may be configured to include a processing circuit such as a DSP or an ASIC instead of or in addition to the CPU 901.

The CPU 901 functions as an arithmetic processing unit and a control unit and controls the overall operation in the information processing device 900 in accordance with various programs. Further, the CPU 901 may be a microprocessor. The ROM 902 stores, for example, an operation parameter and a program used by the CPU 901. The RAM 903 temporarily stores, for example, a program used during execution of the CPU 901 and a parameter appropriately changed in the execution. The CPU 901 may be configured as, in one example, the control unit 130 illustrated in FIG. 4.

The CPU 901, the ROM 902, and the RAM 903 are connected to each other through the host bus 904a including a CPU bus and the like. The host bus 904a is connected, via the bridge 904, to the external bus 904b, an example of which being a peripheral component interconnect/interface (PCI) bus. Moreover, the host bus 904a, the bridge 904, and the external bus 904b are not necessarily configured as a separate component, but their functions may be incorporated into in a single bus.

The input device 906 is implemented as a device allowing the user to input information, such as a mouse, a keyboard, a touch panel, a button, a microphone, a switch, and a lever. In addition, the input device 906 may be a remote controller using infrared ray or other electric waves, or may be externally connected equipment, such as a cellular phone or a PDA, operable in response to the user operation of the information processing device 900. Furthermore, the input device 906 may include an input control circuit or the like which is configured to generate an input signal on the basis of information input by the user using the aforementioned input means and to output the generated input signal to the CPU 901. The user of the information processing device 900 may input various types of data to the information processing device 900, or may instruct the information processing device 900 to perform a processing operation, by the user operation of the input device 906.

The output device 907 is configured as a device capable of performing visual or auditory notification of the acquired information to the user. An example of such device includes a display device such as CRT display devices, liquid crystal display devices, plasma display devices, EL display devices, and lamps, a sound output device such as loudspeakers and headphones, and a printer device. The output device 907 outputs, for example, results acquired by various processes performed by the information processing device 900. Specifically, the display device visually displays results acquired by various processes performed by the information processing device 900 in various formats such as text, images, tables, and graphs. On the other hand, the sound output device converts audio signals composed of reproduced sound data, audio data, and the like into analog signals and audibly outputs the analog signals.

The storage device 908 is a device for data storage configured as an example of a storage unit of the information processing device 900. In one example, the storage device 908 is implemented as a magnetic storage device such as an HDD, a semiconductor storage device, an optical storage device, a magneto-optical storage device, or the like. The storage device 908 may include a storage medium, a recording device for recording data on the storage medium, a reading device for reading data from the storage medium, a deletion device for deleting data recorded on the storage medium, and the like. The storage device 908 stores programs and various types of data executed by the CPU 901, various types of data acquired from the outside, and the like. The storage device 908 may be configured as, for example, the storage unit 120 illustrated in FIG. 4.

The drive 909 is a reader-writer for storage media and is included in or externally attached to the information processing device 900. The drive 909 reads the information recorded on a removable storage medium such as a magnetic disc, an optical disc, a magneto-optical disc, or a semiconductor memory mounted thereon and outputs the information to the RAM 903. In addition, the drive 909 can write information on the removable storage medium.

The connection port 911 is an interface connected with external equipment and, for example, is a connection port with the external equipment that can transmit data through a universal serial bus (USB) and the like. According to the embodiment, the connection port 911 may be connected with the camera 300 illustrated in FIG. 4, for example.

The communication device 913 is, for example, a communication interface configured as a communication device or the like for connection with a network 920. The communication device 913 is, for example, a communication card or the like for a wired or wireless local area network (LAN), long term evolution (LTE), Bluetooth (registered trademark), or wireless USB (WUSB). In addition, the communication device 913 may be a router for optical communication, a router for asymmetric digital subscriber line (ADSL), various communication modems, or the like. In one example, the communication device 913 is capable of transmitting and receiving signals and the like to and from the Internet or other communication equipment, for example, in accordance with a predetermined protocol of TCP/IP or the like. The communication device 913 may be configured as, for example, the communication unit 110 illustrated in FIG. 4.

Moreover, the network 920 is a wired or wireless transmission path of information transmitted from a device connected to the network 920. In one example, the network 920 may include a public circuit network such as the Internet, a telephone circuit network, and a satellite communication network, various local area networks (LANs) including Ethernet (registered trademark), a wide area network (WAN), and the like. In addition, the network 920 may include a dedicated circuit network such as an internet protocol-virtual private network (IP-VPN).

An example of the hardware configuration capable of implementing the functions of the information processing device 900 according to the present embodiment is illustrated above. The respective components described above may be implemented using universal members, or may be implemented by hardware that is specific to the functions of the respective components. Accordingly, it is possible to change a hardware configuration to be used appropriately depending on the technical level at each time of carrying out the embodiments.

Moreover, a computer program for implementing each of the functions of the information processing device 900 according to the present embodiment may be created, and may be mounted in a PC or the like. Furthermore, a computer-readable recording medium on which such a computer program is stored may be provided. The recording medium is, for example, a magnetic disc, an optical disc, a magneto-optical disc, a flash memory, or the like. The computer program may be distributed, for example, through a network without using the recording medium.

<<6. Summary>>

One embodiment of the present disclosure is described in detail above with reference to FIGS. 1 to 11. As described above, the information processing device 100 acquires the sensor information from the plurality of sensor devices 200 that measure the pressure distribution of the attached part of the body of the user, and estimates the arrangement of the plurality of parts to which the sensor devices 200 are attached on the basis of the captured image including the plurality of parts. Then, the information processing device 100 calculates information on the plurality of parts from the acquired sensor information on the basis of the estimated arrangement of the plurality of parts. This makes it possible for the information processing device 100 to obtain useful information such as ZMP of both feet in which the attitude of the target person is taken into consideration from the sensor information.

In one example, the sensor device 200 may be an insole type. In this case, the information processing device 100 can calculate the ZMP of both feet without use of the force platform. In addition, for the action in which the foot moves away from the ground like swing motion of golf, the information processing device 100 can obtain information other than the pressure distribution, such as the attitude of both feet.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

In one example, an example in which the information processing device 100 is implemented as a smartphone is described in the above embodiment, but the present technology is not limited to this example. In one example, the information processing device 100 may be implemented as any device such as a tablet terminal, a PC, or a server on a network.

Furthermore, the information processing device 100 may be implemented as a single device, or may be partially or entirely implemented as a separate device. In one example, in the function configuration example of the information processing device 100 illustrated in FIG. 4, the storage unit 120 and the control unit 130 may be not necessarily included in a server or the like connected to the communication unit 110 via a network or the like. In addition, the information processing device 100 may be integrally formed with the sensor device 200 or the camera 300.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.

(1) An information processing device including:

an acquisition unit configured to acquire information indicating a measurement result from a plurality of sensor devices configured to measure a pressure distribution of an attached part of a body of a user;
an estimation unit configured to estimate arrangement of a plurality of parts to which the sensor devices are attached on the basis of a captured image including the plurality of parts; and
a calculation unit configured to calculate information on the plurality of parts from the information indicating the measurement result acquired by the acquisition unit on the basis of the arrangement of the plurality of parts estimated by the estimation unit.

(2) The information processing device according to (1),
in which the estimation unit estimates arrangement of the plurality of sensor devices on the basis of an estimation result of the arrangement of the plurality of parts.
(3) The information processing device according to (1) or (2),
in which the calculation unit calculates zero moment point (ZMP) in all the plurality of parts.
(4) The information processing device according to any one of (1) to (3),
in which the plurality of parts are both feet of the user.
(5) The information processing device according to (4),

in which the sensor device is an insole type sensor.

(6) The information processing device according to any one of (1) to (3),

in which the plurality of parts are both hands of the user.

(7) The information processing device according to any one of (1) to (6),

in which the estimation unit estimates the arrangement of the plurality of parts by estimating a position and an attitude of each of markers provided at the plurality of parts included in the captured image.

(8) The information processing device according to any one of (1) to (7),

in which the estimation unit estimates the arrangement of the plurality of parts by performing image recognition on the plurality of parts included in the captured image.

(9) The information processing device according to any one of (1) to (8),

in which the estimation unit repeatedly estimates depending on actions of the plurality of parts.

(10) The information processing device according to any one of (1) to (9),

in which the acquisition unit acquires the information indicating the measurement result transmitted from the sensor device.

(11) The information processing device according to any one of (1) to (9),

in which the acquisition unit acquires the information indicating the measurement result from display contents displayed on display devices provided at the plurality of parts included in the captured image.

(12) An information processing method executed by a processor, the information processing method including:

acquiring information indicating a measurement result from a plurality of sensor devices configured to measure a pressure distribution of an attached part of a body of a user;

estimating arrangement of a plurality of parts to which the sensor devices are attached on the basis of a captured image including the plurality of parts; and

calculating information on the plurality of parts from the acquired information indicating the measurement result on the basis of the estimated arrangement of the plurality of parts.

Reference Signs List

1       sensing system

100     information processing device

110     communication unit

120     storage unit

130     control unit

131     acquisition unit

133     estimation unit

135     calculation unit

200     sensor device

300     camera

**Claims**

1.  An information processing device comprising:

an acquisition unit configured to acquire information indicating a measurement result from a plurality of sensor devices configured to measure a pressure distribution of an attached part of a body of a user;

an estimation unit configured to estimate arrangement of a plurality of parts to which the sensor devices are attached on the basis of a captured image including the plurality of parts; and

a calculation unit configured to calculate information on the plurality of parts from the information indicating the measurement result acquired by the acquisition unit on the basis of the arrangement of the plurality of parts estimated by the estimation unit.

2. The information processing device according to claim 1,
wherein the estimation unit estimates arrangement of the plurality of sensor devices on the basis of an estimation result of the arrangement of the plurality of parts.

3. The information processing device according to claim 1,
wherein the calculation unit calculates zero moment point (ZMP) in all the plurality of parts.

4. The information processing device according to claim 1,
wherein the plurality of parts are both feet of the user.

5. The information processing device according to claim 4,
wherein the sensor device is an insole type sensor.

6. The information processing device according to claim 1,
wherein the plurality of parts are both hands of the user.

7. The information processing device according to claim 1,
wherein the estimation unit estimates the arrangement of the plurality of parts by estimating a position and an attitude of each of markers provided at the plurality of parts included in the captured image.

8. The information processing device according to claim 1,
wherein the estimation unit estimates the arrangement of the plurality of parts by performing image recognition on the plurality of parts included in the captured image.

9. The information processing device according to claim 1,
wherein the estimation unit repeatedly estimates depending on actions of the plurality of parts.

10. The information processing device according to claim 1,
wherein the acquisition unit acquires the information indicating the measurement result transmitted from the sensor device.

11. The information processing device according to claim 1,
wherein the acquisition unit acquires the information indicating the measurement result from display contents displayed on display devices provided at the plurality of parts included in the captured image.

12. An information processing method executed by a processor, the information processing method comprising:

acquiring information indicating a measurement result from a plurality of sensor devices configured to measure a pressure distribution of an attached part of a body of a user;

estimating arrangement of a plurality of parts to which the sensor devices are attached on the basis of a captured image including the plurality of parts; and

calculating information on the plurality of parts from the acquired information indicating the measurement result on the basis of the estimated arrangement of the plurality of parts.

# FIG. 1

# FIG. 2

# FIG. 3

# FIG. 4

1

100

**INFORMATION PROCESSING DEVICE**

130

**CONTROL UNIT**

131
**ACQUISITION UNIT**

200
**SENSOR DEVICE**

110
**COMMUNICATION UNIT**

133
**ESTIMATION UNIT**

120
**STORAGE UNIT**

300
**CAMERA**

135
**CALCULATION UNIT**

# FIG. 5

200A    20A          20B    200B

300

100

# FIG. 6

20A                                                            20B

# FIG. 7

# FIG. 8

34A

33B    33A

# FIG. 9

# FIG. 10

```
        START
          │
          ▼
┌─────────────────────────┐
│ INITIALIZATION PROCESSING│──── S102
└─────────────────────────┘
          │
          ▼
┌─────────────────────────┐
│ ACQUIRE SENSOR INFORMATION│──── S104
└─────────────────────────┘
          │
          ▼
┌─────────────────────────┐
│   ACQUIRE CAPTURED IMAGE │──── S106
└─────────────────────────┘
          │
          ▼
┌─────────────────────────┐
│ ESTIMATE POSITION AND ATTITUDE │──── S108
│        OF AR MARKER      │
└─────────────────────────┘
          │
          ▼
┌─────────────────────────┐
│   ESTIMATE ARRANGEMENT   │──── S110
│     OF SENSOR DEVICE     │
└─────────────────────────┘
          │
          ▼
┌─────────────────────────┐
│ CALCULATE ZMP OF BOTH FEET│──── S112
└─────────────────────────┘
          │
          ▼
    NO  ◇ TERMINATION? ◇──── S114
  ┌─────
  │       │ YES
  │       ▼
  │  ┌─────────────────────────┐
  │  │  TERMINATION PROCESSING  │──── S116
  │  └─────────────────────────┘
  │       │
  │       ▼
  │      END
```

## FIG. 11

## INTERNATIONAL SEARCH REPORT

| | International application No. |
|---|---|
| | PCT/JP2016/052296 |

**A. CLASSIFICATION OF SUBJECT MATTER**
*A61B5/11*(2006.01)i

According to International Patent Classification (IPC) or to both national classification and IPC

**B. FIELDS SEARCHED**

Minimum documentation searched (classification system followed by classification symbols)
A61B5/11

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2013-192721 A (Terumo Corp.), | 1-2,4-12 |
| Y | 30 September 2013 (30.09.2013), claim 1; paragraphs [0012], [0034], [0038], [0049] (Family: none) | 3 |
| Y | JP 2009-285269 A (Hirofumi SHINOZAKI), 10 December 2009 (10.12.2009), paragraphs [0024] to [0028] (Family: none) | 3 |
| Y | JP 2008-48981 A (Kochi University of Technology), 06 March 2008 (06.03.2008), paragraphs [0059] to [0066] (Family: none) | 3 |

☐ Further documents are listed in the continuation of Box C.     ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 07 April 2016 (07.04.16) | 26 April 2016 (26.04.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
|---|---|

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012120648 A

**Non-patent literature cited in the description**

- **HIROKAZU KATO ; MARK BILLINGHURST ; KOICHI ASANO ; KEIHACHIRO TACHIBANA.** An Augmented Reality System and its Calibration based on Marker Tracking. *TVRSJ,* 1999, vol. 4 (4